# DEMANDE DE BREVET EUROPEEN

(11) **EP 0 214 909 A1**
(43) Date de publication de la demande: **18.03.1987**
(21) Numéro de dépôt: 86401933.6
(22) Date de dépôt: 02.09.1986
(51) Int. Cl.: C07K 17/00, G01N 33/552, G01N 33/545, G01N 33/558, C12Q 1/68, G01N 27/26

(54) **Moyens et procedes de transfert de proteines et/ou d'acides nucleiques sur une surface receptrice supportee**

(30) Priorité: 02.09.1985 FR 8513046; 01.10.1985 FR 8514579
(71) Demandeur: PLANT GENETIC SYSTEMS N.V., B-1040 Bruxelles (BE)
(72) Inventeur: Vandekerckhove, Joel Stefaan, B-8021 Loppem (BE)
(74) Mandataire: Gutmann, Ernest

(57) **Abrégé**

L'invention concerne des moyens pour la réception et la retenue sur une surface supportée de protéines et/ou d'acides nucléiques.

L'invention consiste à amener à l'état solubilisé des protéines et/ou des acides nucléiques au contact d'une surface libre formée sur un support chimiquement inerte, ladite surface possédant un caractère généralement hydrophobe tout en portant des charges d'un signe déterminé, en présence d'un détergent portant des charges d'un signe opposé au signe déterminé, la concentration du détergent étant suffisante pour maintenir, si besoin, la solubilité desdites protéines et/ou des acides nucléiques, mais ne dépassant pas celle qui entraînerait une fixation trop sélective du détergent à ladite surface, au détriment des protéines et/ou des acides nucléiques.

## Description

L'invention concerne des moyens pour la réception et la retenue sur une surface supportée, de préférence celle d'un support poreux, de protéines et/ou d'acides nucléiques tels que des ARN ou ADN qui y sont amenées et ce notamment par des techniques de transfert à partir de solutions les contenant, et amenées au contact de cette surface.

Pour la commodité du langage, l'expression "protéines" est utilisée dans ce qui suit pour désigner non seulement les protéines selon l'acception générale de ce terme y compris les protéines antigéniques, mais également tous autres polypeptides, lipoprotéines, gly- co-protéines, fragments de protéines, polymères organiques ou polyglycanes. L'invention s'applique plus particulièrement à la fixation et à l'immobilisation de une ou plusieurs protéines sur la susdite surface supportée, après leur transfert à partir d'une plaque ou feuille de gel, par exemple à base de polyacrylamide. On sait que de telles plaques ou feuilles de gel sont couramment utilisées, par exemple pour fractionner un mélange de protéines par électrophorèse.

Diverses techniques ont naturellement déjà été décrites pour réaliser de tels transferts. Ceux-ci sont souvent nécessaires, en particulier lorsque des opérations ultérieures destinées à être réalisées sur ces protéines ne peuvent l'être sur le gel lui-même. C'est notamment pour remédier à de telles difficultés que des techniques de transfert des protéines sur des surfaces plus favorables ont été mises au point au cours de ces dernières années.

Une telle surface est avantageusement celle d'une feuille de nitrocellulose dont avait été reconnue la capacité de fixer des protéines par adsorption à sa surface.

D'une façon générale, ces techniques de transfert comprennent la mise en contact de la feuille ou plaque de gel contenant les protéines avec une feuille ou filtre de nitrocellulose et la réalisation d'une "élution" visant à faire passer les protéines de la feuille de gel à la feuille de nitrocellulose.

Cette élution peut être réalisée par diverses techniques, par exemple par diffusion ou électroélution à l'aide d'une solution tamponnée, sous l'influence d'un champ électrique, concernant des techniques courantes on peut, par exemple, se référer à l'article général intitulé "Protein Blotting : Principles and Applications" (Transfert par absorption de protéines : principes et applications) de Jonathan M. Gershoni et al. publié dans Analytical Biochemistry 131, 1-15 (1983).

Pour que ce transfert puisse s'effectuer dans de bonnes conditions, les protéines doivent en général se trouver dans un état solubilisé ou solubilisable.

Une telle condition s'avèrait naturellement déjà nécessaire à l'occasion de la séparation des protéines contenues dans un mélange, notamment par électrophorèse dans un gel du type sus-indiqué. C'est la raison pour laquelle il est courant d'avoir recours à un détergent, tel que le dodécylsulfate de sodium (S D S).

Il existe d'ailleurs sur le marché des feuilles de gel de polyacrylamide déjà chargées de S D S, prêtes à l'emploi pour l'électrophorèse en présence d'un tampon approprié.

Les applications de cette technique sont nombreuses. En particulier, les feuilles de nitrocellulose sur lesquelles avaient été transférées les protéines, peuvent être amenées au contact de solutions de réactifs ayant une affinité particulière pour l'une d'entre elles, la réaction résultant de cette affinité pouvant ensuite être détectée par des moyens classiques. Il en est ainsi par exemple, dans le cas où, parmi les protéines transférées, il existe un antigène dont la présence dans le mélange initial de protéines était à détecter. Le réactif utilisé dans ce dernier cas peut alors être constitué par un anticorps ou une lectine. Ce réactif porte lui-même un marqueur ou peut à son tour, être détecté par une autre molécule affine marquée.

Si les feuilles de nitrocellulose sont aujourd'hui encore parmi les plus utilisées, il en existe également d'autres ; par exemple, des papiers auxquels sont couplés de façon covalente, des groupes de diazo- benzyloxyméthyle, ou encore des membranes de polyhexa- méthylène-adipamine (connues sous la marque NYLON) modifiées par des groupes aminés tertiaires, par exemple ceux connus sous la marque ZETABIND. La séparation des différentes protéines d'un mélange se révèle aussi souvent nécessaire dans le cas où l'on veut procéder à une analyse de la structure primaire de certaines d'entre elles : une technique couramment utilisée peut mettre en jeu les techniques de dégradation type "dégradation d'Edman", dont le principe est rappelé, par exemple, dans un article de Michael W. HUNKAPILLER et al. intitulé "Protein Séquence Analysis automated Microse- quencing" (Analyse de la séquence de protéines de micro- séquençage automatisé), Science, vol. 219, Fév. 1983.

Dans ce dernier type d'application, des problèmes supplémentaires se posent surtout dans les opérations de séquençage qui doivent être effectuées sur des protéines qui ont, au préalable, été séparées dans un gel de polyacrylamide, et ce, tout particulièrement en présence de dodécylsulfate de sodium.

En effet, les opérations de séquençage selon le principe rappelé ci-dessus, impliquent des traitements de ces protéines par des solutions organiques ou fortement acides, par exemple avec une solution ou des vapeurs d'acide chlorhydrique 6N, à des températures de 110' nécessaires pour hydrolyser la protéine ou avec une solution ou des vapeurs d'acide trifluoroacétique concentré, à des températures de 55'C, nécessaires pour hydrolyser les bandes de protéines et séparer les extrémités N-terminales.

Ces opérations ne peuvent naturellement pas être réalisées sur les parties du gel retenant la fraction de protéines à étudier. Elles ne peuvent l'être davantage sur la fraction de protéines transférées sur des feuilles ou membranes du type sus-indiqué. Ces dernières sont en effet trop fragiles pour résister aux réactions chimiques mises en oeuvre dans les dégradations d'Edman.

D'une façon générale, il a donc été nécessaire jusqu'à ce jour, d'extraire les protéines à séquencer de la feuille de gel les contenant et, en particulier lorsqu'on avait eu recours à un gel chargé en détergents, de séparer ces derniers des protéines extraites, notamment par dialyse ou des techniques d'extraction ou de précipitation.

La manipulation de quantités souvent extrêmement faibles de protéines extraites dans les conditions sus-indiquées, est souvent très difficile, même si les techniques et les appareillages utilisés à cet effet (voir par exemple l'article de Michael W. HUNKAPILLER et al. déjà mentionné ci-dessus) ont été l'objet de perfectionnements constants.

En particulier, il a déjà été proposé, pour éviter les extractions parasites de protéines dans les séquençeurs, d'introduire ces protéines à l'état préalablement solubilisées dans une solution de sels d'ammonium quaternaire polymériques dénommés polymétho-_ bromure de 1,5 diméthyl - 1,5 diazaundécaméthylène.

Ce composé également connu sous la marque POLYBRENE est alors capable de former sur les surfaces en verre du récipient une pellicule à laquelle le polypeptide s'incorpore de sorte qu'il est ensuite protégé de l'action mécanique des flux de solvants utilisés pour les lavages.

La pellicule est aisément pénétrable par les réactifs d'Edman. Les réactions de dégradation nécessaires à la réalisation du séquençage peuvent être alors aisément réalisées.

Ce produit dont l'utilisation aux fins qui précèdent, a pour la première fois été décrit par G.E. TARR et al. anal. Biochem 75.621 (1976) sera par la suite désigné par la marque sous laquelle il est commercialisé, à savoir POLYBRENE pour de simples raisons de commodité de langage.

Il va naturellement de soi, que l'utilisation de ce terme dans le cadre de cette demande de brevet, n'a pas vocation à porter atteinte aux droits du ou des titulaires de la marque POLYBRENE.

Si ce produit a permis le séquençage de quantités encore plus faibles qu'auparavant de protéines, notamment de l'ordre de 200 pmoles, les problèmes relatifs aux difficultés d'obtention des protéines séparées sur gel dans un état tel que l'on puisse ensuite envisager les opérations de séquençage, n'ont toujours pas encore reçu de solution pratique.

Le but de l'invention est de remédier en grande partie à ces divers inconvénients, particulièrement de fournir des moyens permettant le transfert directement et l'immobilisation de protéines, par exemple à partir d'un gel de polyacrylamide, sur une surface libre formée sur un support, ces protéines immobilisées pouvant alors être directement étudiées ou analysées dans les séquençeurs à phase gazeuse, par exemple comme décrit par HEWICK, R.M. HUNKAPILLER, M.W. HOOD, L.E. et DREYER, W.J. (1981) J. BIOL. CHEM. 256, 7990-7997.

Le procédé selon l'invention de transfert et d'immobilisation de protéines sur une surface libre formée sur un support chimiquement inerte, de préférence poreux, à partir d'un milieu susceptible de les fournir, amené en contact avec cette surface, se caractérise en ce que ladite surface, de préférence initialement exempte de protéines et/ou d'acides nucléiques, a un caractère généralement hydrophobe tout en portant des charges d'un signe déterminé et en ce que les protéines et/ou les acides nucléiques sont amenés à l'état solubilisé au contact de ladite surface, en présence d'un détergent portant des charges de signe opposé au signe déterminé, la concentration du détergent étant suffisante pour, si besoin, maintenir la solubilité desdites protéines et/ou desdits acides nucléiques dans le milieu, mais ne dépassant pas celle qui entrainerait une fixation trop sélective du détergent à ladite surface au détriment des protéines et/ou des acides nucléiques.

L'invention découle en effet de la découverte qu'en opérant dans les conditions qui précèdent, le détergent mis en oeuvre paraissait agir comme véhicule de transfert de la protéine sur la surface libre ainsi formée, l'excès de détergent pouvant ensuite être aisément lavé sans que les protéines transférées soient détachées de la surface libre.

Dans la définition qui précède du procédé selon l'invention, il importe de préciser quelques unes des notions évoquées.

L'expression "chimiquement inerte" se rapporte à tout support capable de résister aux solvants aqueux et organiques couramment utilisés dans les techniques de séquençage et d'hydrolyse acide-rappelées plus haut, et également aux températures couramment utilisées.

En particulier, on peut avoir recours pour la constitution de ce support à tout matériau, de préférence poreux, résistant à des solutions d'acide chlorhydrique 6N, à une température de 110°C pendant 24 heures.

Un matériau approprié, répondant à cette condition, est constitué par le verre.

De façon non limitative, le support sera constitué de fibre de verre.

D'autres matériaux sont également utilisables en tant que supports pour la mise en oeuvre du procédé de l'invention.

On citera, à titre d'exemples non limitatifs, des polymères de l'acide méthacrylique de formule I ci-après : 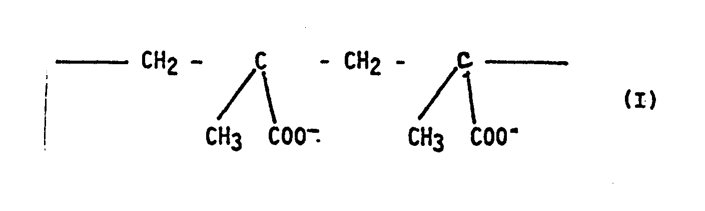 des copolymères de l'acide méthacrylique et de l'étₕy-lène de formule II ci-après : 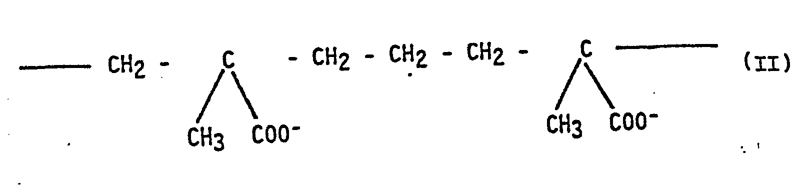 des copolymères de chlorure de vinyle et de l'acétate de vinyle de formule III ci-après : 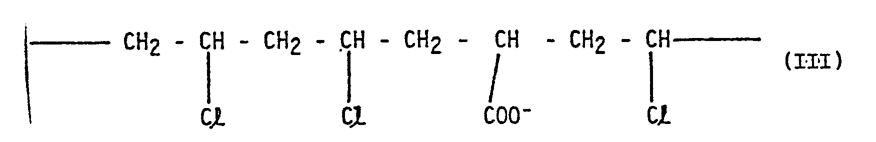 des polymères de l'acide cis-oléique de formule IV ci-après :

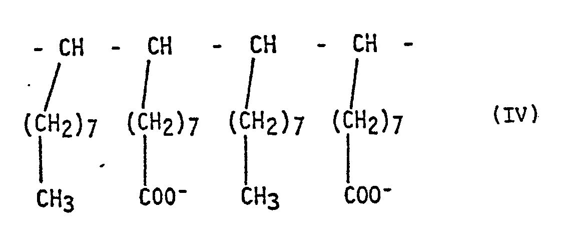

De même l'expression "surface du support" n'exclut pas, surtout lorsque le support est poreux, que les protéines puissent pénétrer à l'intérieur de ce support. En fait, une telle pénétration permet la fixation, s'il y a lieu, de quantités plus importantes de protéines que ne le permettrait une surface lisse et leur meilleure immobilisation. Il est cependant clair que "macroscopiquement", l'on se trouve toujours en présence d'une surface, surtout lorsque ce support se présente sous la forme d'une feuille ou d'une membrane.

Il n'est guère nécessaire d'expliciter le terme "hydrophobe" dont l'acception générale ne pose pas de problème aux chimistes. D'une façon générale, une surface ou un groupe hydrophobe présentent un caractère répulsif vis-à-vis de l'eau.

De même, il ne faut pas nécessairement prendre à la lettre l'expression faisant apparaitre : "les protéines sont amenées à l'état solubilisé au contact de ladite surface en présence d'un détergent..." Là encore, il s'agit d'un phénomène apparent. Il ne peut être exclu qu'il ne se produise au contact immédiat de la surface libre, une précipitation de complexes entre le détergent et les protéines, au moment même de leur transfert. On ne peut en particulier, exclure l'intervention de ces complexes dans l'effet d'immobilisation observé desdites protéines.

De même, l'Homme du Métier est à même de régler les intervalles de concentration de détergents utilisables pour répondre à la condition correspondante exprimée dans la définition générale sus-indiquée de l'invention. Ces concentrations relatives peuvent dépendre de la nature du détergent utilisé. Lorsque le transfert est effectué à partir d'un gel, à la suite d'une opération de fractionnement de protéines par électrophorèse, comme dans les cas préférés qui seront évoqués plus loin, la teneur initiale en détergent du gel lui-même sera également généralement suffisante pour assurer le transfert et l'immobilisation de ces protéines à la surface libre du support.

Dans une première variante de l'invention, et surtout lorsque le détergent utilisé sera constitué par du SDS, la surface libre du support devra comporter des groupements porteurs de charges positives.

Dans ce cas, l'invention mettra généralement en oeuvre le produit nouveau que représentent les pellicules immobilisées, formées à la surface du support chimiquement inerte par l'intermédiaire de liaisons assurant leur insolubilisation vis-à-vis de solutions aqueuses et organiques, ladite pellicule étant formée d'un produit initialement dépourvu de protéines, et comportant à la fois des groupes hydrophobes et des groupes chargés positivement affleurant à sa surface libre.

Dans le cas où le support est formé en verre, la matière dont est constituée la pellicule est avantageusement formée par un polymère hydrophobe, ou des mélanges de différentes polybases comprenant des chaînes hydrocarbonées, dans lesquelles sont intercalés des groupements fournissant des charges positives, notamment des groupes d'ammonium quaternaire dont une partie au moins affleure à la surface libre, et l'autre partie coagit avec les groupes silanol existants à la surface du support de verre pour assurer la fixation par liaisons ioniques de la pellicule à la surface du verre.

Ce polymère est avantageusement formé par le POLYBRENE.

Dans une variante de mise en oeuvre de l'invention, le support de verre est "pré-activé" dans le but d'obtenir un nombre plus grand de groupes silanol affleurant à sa surface.

Ce faisant, la capacité de fixation du polymère sur le support, et la quantité de protéines immobilisées ultérieurement sur ce support seront plus importantes.

La pré-activation du support peut être réalisée par addition d'acide trifluoroacétique concentré en solution à des températures variant d'environ 25⁰C à environ 50'C, pendant une durée d'environ 30 mn à environ 8 heures.

La pré-activation du support de fibre de verre peut être également réalisée par addition d'acide nitrique concentré en solution, à température ambiante, pendant une durée variant d'environ une à environ huit heures.

L'invention met donc à profit la capacité d'une pellicule continue à la surface du verre à former des liaisons ioniques avec des groupes silanol négativement chargés affleurant à la surface du verre poreux, et le cas échéant, à masquer des groupes silanols n'intervenant pas dans la réaction de fixation.

La réalisation de ces liaisons ioniques suffit à insolubiliser une pellicule au moins monomoléculaire de POLYBRENE à la surface de verre vis-à-vis des solutions aqueuses (le POLYBRENE est soluble dans l'eau) tout en préservant sa capacité à absorber des protéines même en présence d'un détergent, de charge négative, en particulier de SDS.

On rappelle que le POLYBRENE est un polymère dont la structure peut être représentée par la formule V ci-après : 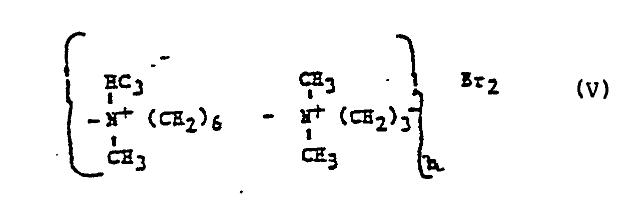 dans laquelle on peut varier dans de grandes proportions. On appréciera que la pellicule peut également être constituée à partir de polymères semblables, dans lesquels les groupes triméthylène et hexaméthylène séparant les groupes ammonium quaternaire, peuvent être remplacés par des chaînes plus courtes ou plus longues, comprenant par exemple de 2 à 10 motifs méthylène.

Une des conditions à prendre en considération pour le choix de ces polymères sera le nombre d'atomes d'azote intercalés dans les chaînes pour que tous ne soient pas engagés dans les liaisons ioniques formées entre les groupes -0-S-03 du verre et les groupes -^{CH}₂-N⁺(CH₃)₂-CH₂- du polymère.

Les indications qui précèdent ne sont pas limitatives en ce qui concerne les types de polymères utilisables pour la constitution des pellicules chargées positivement. On peut mentionner le polyéthylénimine, le polyvinyl pyridine à titre d'autres exemples de matières utilisables.

Des polymères préférés de polyvinyl pyridine comprennent les sels de poly (2-vinyl-N-méthylpyridine) de formule VI ci-après, et les sels de poly (4-vinyl-N-méthylpyridine) de formule VII ci-après :

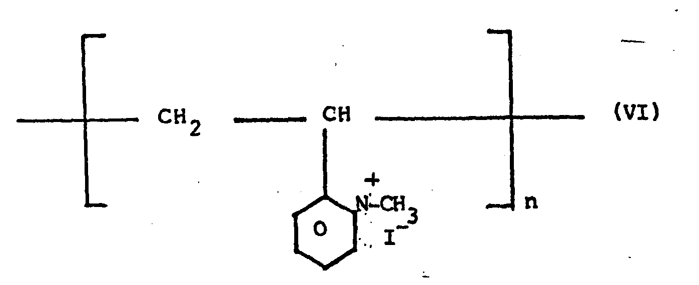

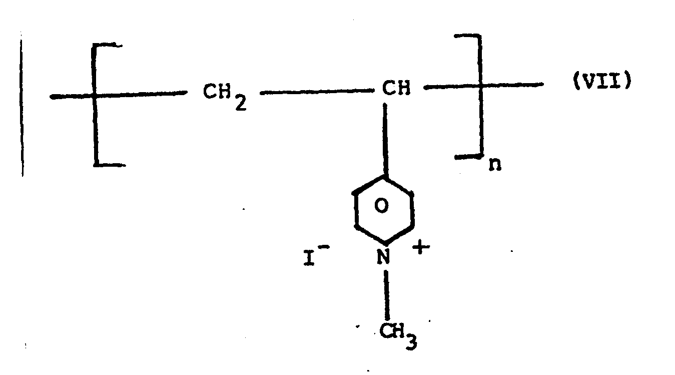

D'une façon générale, la pellicule peut être formée à partir de toute molécule comportant au niveau moléculaire, à la fois des régions hydrophobes, en particulier portées par des éléments de chaines aliphatiques ou aromatiques, et des régions porteuses de charges positives.

Quel que soit le polymère utilisé comportant les groupes ammonium quaternaire nécessaires à la fixation d'une mince pellicule à la surface du support chimiquement inerte, on obtient en définitive une pellicule caractérisée par des ponts hydrophobiques (hexaméthylène et triméthylène respectivement dans le cas de POLYBRENE) comprenant un excès d'ions ammonium quaternaire intercalés entre les ponts. La surface ainsi produite fonctionne à la façon d'un échangeur d'anions forts ayant un caractère hydrophobe. Dans les réalisations qui pré_{c}è- dent on peut naturellement utiliser des détergents autres que le SDS.

D'une façon générale, on peut avoir recours à tout détergent formé de chaînes hydrophobes notamment de nature aliphatique ou aromatique, ces chaînes portant en outre des groupements suffisamment hydrophiles pour les rendre hydrosolubles. Ces détergents doivent en outre, être aptes à favoriser la solubilisation des protéines dans des milieux aqueux.

A titre d'exemples d'autres détergents, on mentionnera l'acide stéarique, l'acide palmitique, le dioxycholate. Avantageusement le milieu à partir duquel des protéines sont transférées à la surface libre de la pellicule est lui-même formé d'un gel de polyacrylamide contenant lui-même du SDS. Le transfert peut alors être réalisé par contact intime entre la feuille de gel et le support portant la susdite pellicule, le transfert étant alors effectué de toute façon appropriée (diffusion, convection, électro-élution, etc).

Lorsque l'on a recours à un solvant tamponné extérieur pour favoriser cette élution, on peut avoir recours à une solution tamponnée classique.

A titre d'exemples non limitatifs de solutions tamponnées, on citera des solutions contenant 50mM de borate de sodium (pH : 8,0), des solutions de transport renfermant du tris-HCl, de la tris-glycine, éventuellement additionnées de méthanol à 20%.

Dans une variante de mise en oeuvre de l'invention où on utilise un support de fibres de verre recouvertes d'iodure de poly (4-vinyl-N-méthylpyridine), un tampon préféré est une solution de borate de tris-(hydroxyméthylamino) méthane (50mM).

₋ Dans le cas où la concentration en SDS du gel est suffisante -à assurer la solubilisation des protéines, il est en général inutile de travailler avec une solution tamponnée contenant elle-même encore une certaine teneur en détergent. La teneur en détergent du gel est en général suffisante pour promouvoir le transfert dans les conditions qui ont été indiquées. Il en est ainsi, en particulier lorsque la teneur du gel de poly- acrylamide en SDS utilisé, pour réaliser l'électrophorèse préalable d'un mélange de protéines, contenait 0,1\ de SDS.

Il est significatif que l'invention tire profit des propriétés à priori contradictoires des différents constituants mis en jeu. Il est en effet rappelé que le POLYBRENE est soluble dans l'eau. Sa fixation sur le support inerte le protège cependant de cette propriété qui autrement pourrait être nuisible, particulièrement si les quantités de solution tamponnée mises en oeuvre pour réaliser l'élution étaient relativement importantes. C'est la raison pour laquelle il n'est pas avantageux d'utiliser un support pourvu à sa surface de POLYBRENE non fixé. Celui-ci pourrait alors à l'occasion du susdit contact capter des protéines qui seraient ensuite perdues, par exemple dans le cas de lavages répétés de la surface libre supportée.

La présence de POLYBRENE non "fixé" pourrait encore être nuisible pour une raison supplémentaire. En effet, le POLYBRENE et le SDS forment des complexes insolubles dans l'eau. Leur formation éventuelle serait alors un obstacle au transfert complet des protéines à partir du gel vers la pellicule supportée. Enfin la fixation du POLYBRENE, sous forme d'une fine pellicule à la surface du support inerte, ne fait pas obstacle au lavage de la surface libre retenant les protéines avec des solvants organiques, y compris ceux qui dissolvent aisément le POLYBRENE ou des polymères chargés analogues.

Bien entendu, dans la technique qui précède, le gel de polyacrylamide peut être remplacé par tout autre gel, par exemple des gels à base d'agarose, de séphadex, de cellulose, etc...

L'invention concerne également un procédé particulièrement simple de fabrication de pellicules supportées répondant aux conditions sus-indiquées. En particulier, le procédé de formation d'une pellicule insolubilisée vis-à-vis de solvants aqueux ou organiques sur un support chimiquement inerteporeux de préférence, et comportant des groupes chargés négativement, est caractérisé par la mise en contact de la surface de ce support avec une solution, aqueuse ou organique, d'une matière dont la structure moléculaire comporte à la fois des régions hydrophobes et des groupes chargés positivement permettant sa solubilisation, le nombre de groupes chargés positivement et la concentration de la matière dans la solution étant suffisante pour permettre la neutralisation des charges négatives portées par le support, ainsi que la formation et la rétention d'une pellicule hydrophobe portant un excédent de charges positives à sa surface, au moins monomoléculaire, après rinçage et séchage dudit support revêtu de la pellicule insolubilisée alors formée. Avantageusement, la matière utilisée est une polybase telle que décrite ci-dessus.

Des solvants organiques appropriés comprennent le méthanol, l'éthanol, le dioxane, la pyridine.

La mise en contact du support et de la polybase peut se réaliser par tout moyen connu, notamment par simple versement de la solution de polybase sur le support, par immersion du support dans une solution de la polybase utilisée pendant une durée variant d'environ 15 minutes à environ 6 heures.

Il appartiendra à l'homme de métier de déterminer, par de simples essais de routine, la durée de contact optimale pour obtenir un revêtement satisfaisant, notamment en fonction des produits utilisés.

Dans l'exemple préféré où l'on utilise en tant que support des fibres de verre et selon une variante de réalisation, les fibres de verre sont cassées par ultrasons de façon à obtenir des microfibres de verre. Ces microfibres sont ensuite mises en suspension dans une solution de la polybase utilisée puis filtrées. Les microfibres recouvertes de la pellicule insolubilisée sont ensuite passées sous presse pour obtenir des feuilles de l'épaisseur désirée.

L'invention concerne également la variante opposée du procédé de transfert et d'immobilisation de protéines qui peut être conçu. Dans cette variante opposée, la surface libre sur laquelle les protéines sont transférées porte alors des groupes chargés négativement et le détergent mis en oeuvre dans l'opération de transfert porte alors des groupes chargés positivement. On peut se retrouver placés dans cette situation lorsque, partant d'une feuille de gel de polyacrylamide dans lequel les protéines sont séparées en la présence d'un détergent inverse (un détergent contenant des charges positives, par exemple le bromure de cétyltriméthylam- monium, commercialisé sous la marque CETAVLON) ou d'une feuille de gel de polyacrylamide initialement chargé en SDS et dans laquelle différentes protéines ont été séparées sous forme de bandes, le gel polyacrylamide SDS est lavé de façon extensive avec une solution contenant 0,5% d'acide acétique et 0,1°s de bromure de cétyltriméthylam- monium. Ce lavage substitue alors au SDS le CETAVLON positivement chargé et transforme le complexe protéines-détergent en des micelles positivement chargées ; le complexe protéines-détergent ainsi formé peut alors être fixé sur un support chimiquement inerte présentant une surface libre hydrophobe, mais portant cette fois-ci des charges négatives. Ces charges négatives peuvent être fournies par une surface de verre, en l'absence de toute pellicule intermédiaire. Les groupes silanol présents à la surface du verre fournissent alors les charges négatives permettant de fixer les groupes triméthylammonium de CETAVLON, tandis que les parties cétylaliphatiques interviennent au niveau de l'interaction entre le détergent et les régions hydrophobes de la surface de verre.

Ceci étant, la surface de verre peut également être pourvue d'une pellicule fixée de façon ionique, covalente ou non (selon des principes analogues à ceux applicables dans le cas précédent), notamment lorsque l'on souhaite renforcer le nombre de charges négatives affleurant à la surface du verre.

La détection des protéines immobilisées selon le procédé de l'invention est effectuée par coloration du support à l'aide d'un colorant. Des colorants appropriés comprennent une solution de fluorescamine dans l'acétone, à des concentrations variant de 1mg/200ml à ₁mg_{/1} ; le cacodylate de fer selon la méthode décrite par MOEREMANS M. et al, Anal. Biochem. 1986, 153, 18-22; l'iodure de 3,3'-dipentyloxacarbocyanine selon la méthode décrite par AEBERSOLD R et al, J. Biol. Chem. 1986, 261, 4229-4238.

En variante, les protéines sont également détectées en exposant le support à une lumière ultraviolette.

Il va de soi que les protéines immobilisées pourront être détectées par tout autre moyen connu en soi, notamment par fluorescence.

Dans ce qui précède, il a surtout été fait référence au transfert des protéines contenues dans une feuille de gel à la surface libre d'un support chimiquement inerte. Il va de soi que le milieu fournissant les protéines peut être de toute autre nature, par exemple consister en une feuille de nitrocellulose ou de NYLON poreux contenant des protéines à l'état adsorbé et qui peuvent en être éluées dans toutes conditions appropriées.

Le procédé de l'invention s'applique particulièrement bien à la détermination de séquences amino-acides partielles des protéines. Le support peut en effet être coupé pour n'en conserver que la portion contenant les protéines immobilisées. Cette portion est ensuite placée dans la chambre de réaction d'un séquençeur à phase gazeuse pour analyser la séquence amino-acide.

Il va de soi que l'invention s'applique également à la détermination de la composition totale en acides aminés de protéines variées.

Dans ce cas, la portion de support contenant les protéines immobilisées est traitée avec de l'acide, notamment HCl 6N ou des vapeurs d'acides. Les acides aminés sont ensuite analysés par les techniques classiques.

Le principe de la présente invention peut également être utilisé pour détecter la présence d'anticorps dans un échantillon biologique.

Dans cette variante d'utilisation, les protéines antigéniques sont fixées sur le support, puis ce dernier est lavé avec une solution de protéines pour saturer les éventuels sites de fixation résiduels du support. La saturation est réalisée avec une solution de protéines non spécifiques, éventuellement un mélange de telles protéines, un sérum total, ou toute combinaison de ces composés, étant entendu que la solution de saturation ne doit pas interférer ou réagir avec les anticorps spécifiques du test de détection.

Le support contenant les protéines antigéniques est mis ensuite en contact avec un échantillon biologique dans lequel la présence d'anticorps est recherchée.

Après lavage pour éliminer les produits de réaction en excès, on met en contact le support avec un second anticorps dirigé contre les premiers anticorps recherchés, ce second anticorps étant couplé à une enzyme telle que la peroxidase ou la phosphatase.

Ce pourra être, par exemple, un anticorps an- ti-lapin de souris dans le cas où l'anticorps recherché est issu de lapin.

La présence de l'anticorps recherché est révélée en ajoutant un réactif, formant un précipité coloré avec l'enzyme couplée au second anticorps. On citera, à titre d'exemples de réactifs utilisables le 4-chloronaphtol avec la peroxidase et un mélange de 5-bromo-4-chloro-3-indolyl-phosphate et de nitro-2-té- trazolium avec la phosphatase.

Le procédé de l'invention s'applique également à la variante inverse où l'on souhaite détecter la présence de protéines antigéniques dans un mélange de protéines, par réaction affine avec les anticorps complémentaires.

Il va de soi que le principe de la présente invention peut également être utilisé pour adsorber toutes sortes de particules présentes dans des micelles de détergent chargé, par exemple des gouttes d'huile, des lipides, sur du verre ou des supports inertes possédant les propriétés décrites ci-dessus.

₋ Il peut également être utilisé pour purifier des détergents contenant des suspensions d'une manière très simple. Une fois saturés, des matériaux absorbés peuvent ensuite être éliminés par des solvants organiques et le support filtrant peut être rechargé en le faisant passer dans une solution d'une polybase.

Des caractéristiques supplémentaires de l'invention apparaitront encore au cours de la description qui suit des conditions dans lesquelles a été réalisé le transfert de protéines diverses à partir de milieux appropriés vers une surface libre supportée par un support inerte répondant aux conditions qui ont été précisées plus haut, ainsi qu'à la lecture des figures qui s'y rattachent dans lesquelles :

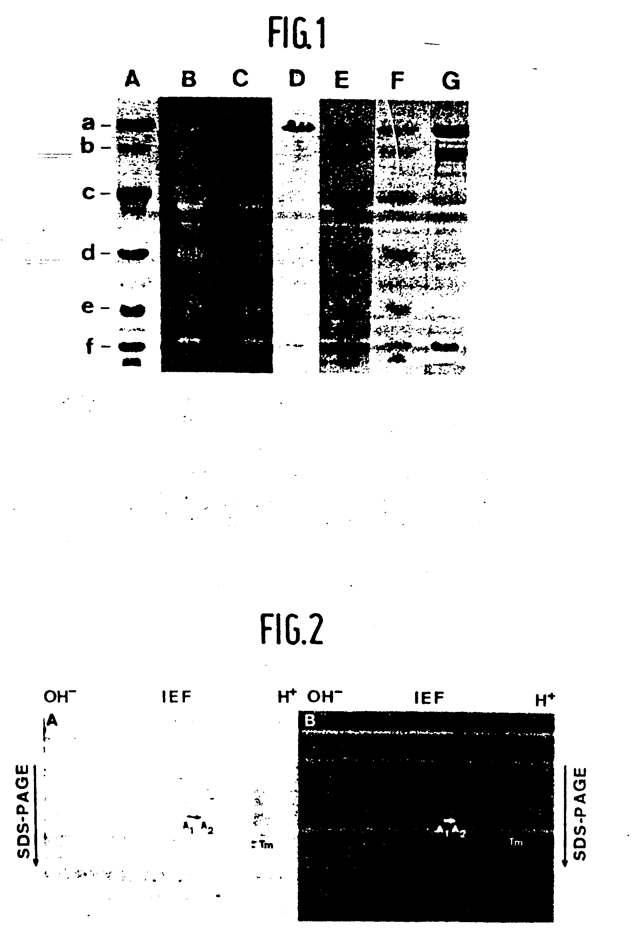

- la figure 1 illustre le principe du transfert de différentes protéines d'un gel de polyacrylamide contenant du S D S sur une fibre de verre recouverte de POLYBRENE.

Le mélange de protéines étalons contient de la phosphorylase b (a) ; de la sérumalbumine bovine (b) ; de l'ovalbumine (c) ; de l'anydrase carbonique (d) ; un inhibiteur de la trypsine du soja (e) ; de la lysozyme (f),

A représente les bandes observées après coloration du gel par le bleu de Coomassie ; B et C, respectivement les taches sur la fibre de verre de la première feuille (_{B}) et de la seconde (C) colorées avec la fluorescamine ; D une coloration avec le bleu de Coomassie des protéines restantes dans le gel, après l'électro- transfert ; E et F respectivement, les taches de protéines après l'électrotransfert sur les première (E) et seconde (F) feuilles des protéines colorées auparavant au bleu de Coomassie ; G les taches de protéines colorées au bleu de Coomassie restantes dans le gel après élimination des taches de transfert colorées au bleu de Coomassie ;
- la figure 2 représente les résultats d'un transfert à deux dimensions de protéines issues de muscle embryonnaire d'un poussin âgé de 18 jours sur une feuille de fibre de verre recouverte de POLYBRENE.

Dans la figure 2, A représente le gel à deux dimensions coloré avec le bleu de Coomassie. Les résultats du transfert des taches de protéines sur le verre recouvert de POLYBRENE sont représentés en B. Les protéines dans B représentent la moitié de la quantité utilisées dans A où A₁, A₂ et Tₘ représentent respectivement la β-actine, a-actine et les tropomyosines. H⁺ et OH⁻ indiquent le côté acide et le côté basique du gel. La séparation par focalisation isoélectrique "IEF" (de l'anglais : isoelectric focussing) a été réalisée dans une direction horizontale, la séparation par électrophorèse sur gel de polyacrylamide contenant du SDS dans une direction verticale.

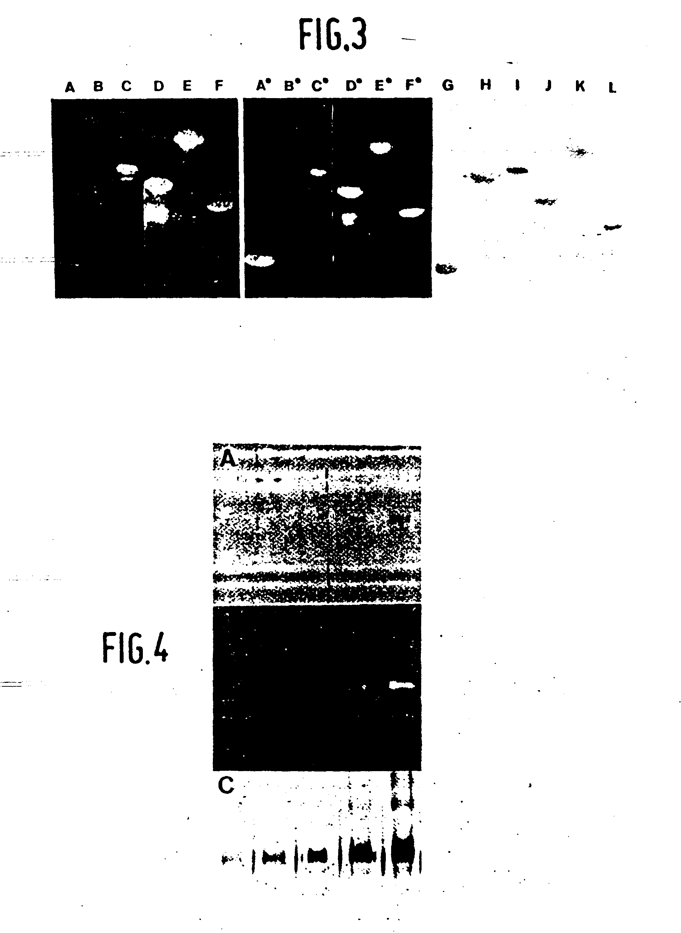

La figure 3 illustre des exemples de transfert de protéines variées : A, B, C, D, E, F représentent la myoglobine, la phosvitine, l'actine, le chymotrypsinogène, la sérumalbumine humaine et la DNase I immobilisés sur la première feuille et colorés avec la fluorescamine. + + + + A , B , C , D , E , F représentent les mêmes taches sur la seconde feuille. G, H, I, J, K, L représentent les mêmes protéines, dans le même ordre, transférées après une coloration dans le gel avec le bleu de Coomassie. Seule la première feuille est montrée ici. Les taches ont été utilisées pour déterminer la capacité de fixation du verre recouvert de _{POL}YBRENE (voir tableau I) et pour comparer leur composition avec celles dérivées de leur séquence connue. - la figure 4 représente la détermination de la sensibilité de l'opération de trtansfert sur fibre de verre. Des quantités variées (10, 50, 100, 500 et 1000 ng) de sérum-albumine humaine ont été utilisées sur un gel de poly-acrylamide à 12,5% et détectées dans le gel avec le bleu de Coomassie (A), la coloration à l'argent (C) la fluorescamine après la fixation sur le POLYBRENE (B).

### MATERIELS ET METHODES

On indique ci-après, les natures des matériels et des méthodes qui ont été utilisés au cours du transfert des protéines.

Le mélange de protéines de référence à bas poids moléculaire a été fourni par Bio Rad Laboratories.

La Sérumalbumine humaine, la DNase pancréatique bovine et la myoglobine de muscle de squelette de Cachalot ont été obtenus de Sigma Chemicals.

L'ovalbumine et le chymotrypsinogène traité au diisopropylfluorophosphate sont des produits WORTHINGTON. La phosvitine d'oeuf de poulet a également été utilisée comme l'une des protéines de référence. L'actine du muscle squelettique de lapin a été préparée à partir de poudres d'acétone selon Spudich et Watt (12). La protéase de Sta^{p}hvlococcus aureus V8 est de MILES, Angleterre.

Les feuilles de fibre de verre (47 x 56 cm) sont des produits WHATMAN, Royaume-Uni.

Le POLYBRENE et la fluorescamine sont des produits de JANSSEN CHIMICA et FLUKA A.G. respectivement. Les produits chimiques utilisés dans le procédé de transfert et les amino-acides ont été utilisés sans autre purification.

Les réactifs et les solvants utilisés dans le séquençage en phase gazeuse ont été fournis par APPLIED BIOSYSTEMS INC. Les solvants utilisés étaient appropriés à l'analyse en chromatographie liquide haute performance (CLHP) des dérivés phénylhydantoine des acides aminés PTH.

### PREPARATION DES FEUILLES DE FIBRE DE VERRE RECOUVERTES DE POLYBRENE -

Des feuilles de papier de microfibres de verre (GD/C) ont été coupées à la taille du gel de polyacrylamide et imprégnées avec une solution de POLYBRENE dans l'eau à 3 mg/ml.

Les feuilles imprégnées ont été séchées à l'air et l'excès de POLYBRENE a été enlevé par lavage avec 100 ml d'eau distillée pendant 10 minutes.

Les feuilles ainsi lavées ont été ensuite utilisées immédiatement, ou stockées dans un milieu sec.

### ELECTROPHORESE SUR GEL DE POLYACRYLAMIDE CONTENANT DU S D S - TRANSFERT ET DETECTION DES PROTEINES IMMOBILI-SEES.

Les échantillons ont été préparés et séparés sur des gels de polyacrylamide contenant du S D S essentiellement selon la méthode décrite par LAEMMLI (13). Les gels étaient épais de 1,5 mm et le pourcentage d'acrylamide utilisé a été de 12,5% sauf indication contraire.

Le transfert électrophorétique a été effectué pendant 20 heures à 3 V/cm et l'ensemble gel-fibre de verre a été assemblé essentiellement selon la méthode décrite pour le transfert de protéines sur de la nitrocellulose (4), la nitrocellulose étant remplacée par deux feuilles de verre recouvertes de POLYBRENE et marquées sur les bords par des encoches, afin de pouvoir réaliser une superposition parfaite après le séchage et la coloration. Le tampon de transport contenait 50 mM de borate de Sodium (pH : 8,0), 20% de méthanol et 0,02% de Bêta-mercaptoéthanol.

Les feuilles de verre recouvertes de POLYBRENE ont été ensuite lavées pendant 10 minutes avec 150 ml de chlorure de sodium à 25 mM et 10 mM de borate de sodium (pH 8,0). Cette opération a été répétée 4 fois et s'est terminée par un lavage à l'eau pendant 5 minutes. Les feuilles ont été séchées pendant la nuit et plongées dans une solution de fluorescamine dans l'acétone frai- chement préparée (de 1 mg/50 ml à 1 mgl/300 ml).

Les protéines ont été visualisées après exposition à l'ultraviolet, avec une lampe à lumière minérale, (Ultraviolet Products, San Gabriel, USA) et photographiées avec un film Polaroid type 665 P/M et un filtre Wratten n" 9, avec des temps d'exposition variant de 1 à 2 minutes.

Dans certaines expériences, les protéines ont été visualisées en premier lieu dans le gel par une coloration pendant 20 minutes avec une solution de Bleu de Coomassie (0,25% de Bleu de Coomassie, 45% de méthanol, 9% d'acide acétique en volume) et décolorées pendant au moins 24 heures avec des solutions chargées plusieurs fois de 5% de méthanol, 7,5% d'acide acétique.

Avant l'électrotransfert, les protéines coloriées ont été redissoutes en introduisant le gel dans 0,1% de SDS, 50 mM de borate de sodium (pH 8,0), et ce tampon a été remplacé plusieurs fois jusqu'à ce que le dernier lavage montre une valeur de pH supérieure à 7,0. Le gel équilibré a été ensuite monté et électrotransféré comme il a été décrit plus haut.

Etant donné que le bleu de Coomassie se lie fortement au POLYBRENE cationique, les protéines ont pu être localisées par la co-électroélution et co-adsorption de la tâche liée à l'origine à la protéine dans le gel.

La dernière procédure sera ici référencée comme l'immobilisation "indirecte" ou "colorée" par le bleu de Coomassie.

### HYDROLYSE ACIDE ET ANALYSE AMINO-ACIDE

La portion de feuille de fibre de verre portant les protéines a été coupée et placée dans un tube Pyrex auquel ont été ajoutés 400 µl d'une solution d'HCl 6 N contenant 0,0005% de f-mercaptoéthanol (14). Les tubes ont été bouchés sous vide et mis à incuber pendant 22 heures. Leurs hydrolysats ont été rapidement séchés, redissous dans 400 µl d'un tampon de citrate de sodium 0,2 M (pH : 2,2) et filtrés.

Les analyses ont été effectuées avec un analyseur d'amino-acides BIOTRONIK équipé d'un détecteur de fluorescence et d'un intégrateur HEWLET PACKARD.

Les acides aminés ont été dosés de façon quantitative après réaction avec du o-phtaldialdéhyde essentiellement de la manière décrite par BENSON et HARE (15).

### DETERMINATION DE LA SEQUENCE AMINO-ACIDE

La portion de feuille en fibre de verre portant les protéines ou les polypeptides immobilisés a été découpée afin de former un disque de 12 mm de diamètre. Les éventuelles bandes de protéines étrangères contami- nantes ont été éliminées et le disque restant a été monté dans la chambre de réaction du séquençeur à phase gazeuse (Applied Biosyst. Inc. USA).

Le séquençeur a été assemblé et utilisé comme décrit par HEWICK et al (16). Les dérivés amino-acides- _{P}TH libérés à chaque étape ont été analysés en utilisant une colonne analytique Cyano-CLHP (IBM Instruments) et le système d'élution décrit par HUNKAPILLER ET HOOD (17). Un système CLHP Waters comprenant 2 pompes M 6000 A, un autoinjecteur WISP 710 B, un système de contrôle M 721 et un détecteur à longueur d'onde fixe M 441 (254 nm) ont été utilisés.

L'obtention d'acides aminés PTH a été mesuré à chaque étape en utilisant un enregistreur à intégration (WATERS DATA Module M 730).

### ELECTROPHORESE SUR GEL DE POLYACRYLAMIDE A DIMENSIONS.

Du tissu embryonnaire de muscle squelettique de poussins âgés de 18 jours a été dissous dans 10% de p-mercaptoéthanol, 3% de dodécylsulfate de sodium, centrifugé et les parties aliquotes contenant des concentrations appropriées de protéines ont été lyophilisées. Le matériel séché a été redissous dans un tampon lyse (18) et les protéines ont été séparées dans un système polyacrylamide à 2 dimensions, comme décrit par Garrels (18).

### DIGESTION DE L'ACTINE AVEC LA PROTEASE DE STAPHYLOCOCCUS AUREUS V8.

Une solution d'actine G (2 mg/ml) dans un tampon salin (2 mM Tris, 0,2 mM ATP, 0,2 M CaCl₂, 0,2 mM β-mercaptoéthanol pH 7,6) a été diluée avec un volume égal de 2 M tris-HCl pH 7,0 et digérée pendant 30 minutes à 25°C avec la protéase V8 (ratio enzyme/substrat : 1/30 en poids). La fragmentation a été terminée par précipitation avec 1/8 en volume d'acide trichloroacétique à 100%.

Le produit obtenu après centrifugation a été redissous dans le tampon (voir ci-dessus) et des parties aliquotes appropriées ont été appliquées sur des gels de polyacrylamide à 20%.

En variante, l'actine G a été diluée (dans les mêmes conditions que ci-dessus), avec un volume égal de 0,1% de dodécylsulfate de sodium, 0,175 M de tris-HCl de pH 7,5 et digérée comme ci-dessus.

La protéolyse a été terminée par l'addition de dodécylsulfate de sodium (concentration finale 2%) et de mercaptoéthanol (concentration finale 10%) (19). Les fragments ont été séparés comme ci-dessus.

### TRANSFERT ET IMMOBILISATION DE PROTEINES VARIEES.

Les résultats obtenus sont exposés dans ce qui suit, en particulier par référence aux figures dont les significations sont rappelées ci-après :
- la figure 1 illustre le principe du transfert de différentes protéines d'un gel de polyacrylamide contenant du S D S sur une fibre de verre recouverte de POLYBRENE.
- La figure 2 représente les résultats d'un transfert à deux dimensions de protéines issues de muscle embryonnaire d'un poussin âgé de 18 jours sur une feuille de fibre de verre recouverte de POLYBRENE.
- La figure 3 illustre des exemples de transfert de protéines variées : A, B, C, D, E, F représentent la myoglobine, la phosvitine, l'actine, le chymotrypsinogène, la sérumalbumine humaine et-la DNase 1 immobilisés sur la première feuille et colorés avec-la fluorescamine. A⁺, B⁺, C⁺, D⁺, E⁺, F⁺ représentent les mêmes taches sur la seconde feuille.

G, H, I, J, K, L représentent les mêmes protéines, dans le même ordre, transférées après une coloration dans le gel avec le bleu de Coomassie. - La figure 4 représente la détermination de la sensibilité de l'opération de transfert sur fibre de verre. Des quantités variées (10, 50, 100, 500 et 1000 ng) de sérum-albumine humaine ont été utilisées sur un gel de polyacrylamide à 12,5% et détectées dans le gel avec le bleu de Coomassie (A), la coloration à l'argent (C) la fluorescamine après la fixation sur le POLYBRENE (B).

### RESULTATS

La grande diversité des protéines susceptibles d'être fixées apparaît à l'examen des résultats ci-après:
- La figure 1 montre les résultats des expériences de transfert effectuées avec le mélange de protéines de référence à bas poids moléculaires fourni par BIO RAD, et séparé sur un gel de polyacrylamide à 12,5%.

Une des encoches du gel a été colorée avec le bleu de Coomassie (fig. 1A) et l'autre a été électro- éluée sur deux feuilles de fibre de verre superposées, recouvertes de POLYBRENE et colorée ensuite avec la fluorescamine (fig. 1 B et 1 C).

La figure 1 D montre un exemple de protéines colorées avec le bleu de Coomassie et restant encore dans le gel après l'opération de transfert.

Les modèles démontrent que, hormis le cas de la phosphorylase b, toutes les protéines standard sont électroéluées quantitativement hors du gel et immobilisées sur plusieurs feuilles de verre recouvertes-de POLYBRENE. En raison de son poids moléculaire élevé, la phosphorylase b n'est pas quantitativement éluée hors du gel et des périodes d'électroélution plus longues semblent nécessaires pour obtenir une élution complète. La quantité de protéines présentes dans chaque bande (environ 10 pg) est suffisante pour saturer la surface correspondante sur la première feuille de verre, après quoi les protéines sont immobilisées sur la seconde feuille. Les protéines présentes seulement en de faibles quantités (qui représentent ici les produits de dégradation des protéines de référence) sont entièrement retenues sur la première feuille.

Les figures 1E et 1F montrent le transfert du même mélange de protéines sur deux feuilles de verre recouvertes de POLYBRENE superposées. Elles ont été auparavant colorées avec le bleu de Coomassie (voir fig. 1A) et redissoutes ensuite en incubant le gel dans le tampon contenant du SDS avant l'électrotransfert. Les protéines restantes dans le gel après cette opération de transfert ont été recolorées avec le bleu de Coomassie et le résultat est montré à la fig. 1F.

Cette expérience montre que les protéines qui ont été auparavant colorées dans le gel peuvent aussi être transférées-et immobilisées sur le verre.

Toutefois, ce transfert "indirect" apparaît être moins quantitatif et plus dépendant du type de protéines que dans le transfert direct.

Non seulement les protéines à poids moléculaire élevé (phosphorylase b et sérumalbumine bovine), mais également des protéines plus petites comme la lysozyme, sont retenues partiellement sur le gel original.

Ici également, les protéines sont immobilisées à des niveaux de saturation sur la première feuille et ensuite adsorbées sur les couches suivantes.

Dans le but de démontrer que la technique de transfert sur verre peut être appliquée avec une large variété de protéines, un extrait de protéines d'un muscle de patte de poulet embryonnaire âgé de 18 jours a été séparé par une électrophorèse classique à deux dimensions sur gel de polyacrylamide. -
- La figure 2A montre les taches de protéines après coloration du gel avec le bleu de Coomassie.
- La figure 2B montre une coloration avec la fluorescamine des taches sur la fibre de verre effectuée à partir d'une séparation similaire et avec la moitié de l'extrait de protéines utilisé pour la figure 2A.

La tache sur le verre apparait comme une copie presque exacte de la protéine visualisée dans le gel.

Les taches principales sont dues à l'actine a et à l'actine et aux isoformes de la tropomyosine. Elles représentent environ 0,5 pg de protéines ainsi qu'il a été déterminé par l'analyse des amino-acides des taches immobilisées (voir ci-dessous). Ces quantités correspondent respectivement à 12 et 17 pmol d'actine et de tropomyosine, malgré l'extrême petitesse des quantités fixées (dans le domaine des picomoles), les feuilles de verre revêtues selon l'invention permettent déjà l'analyse d'au moins certains acides aminés éluant avec le premier tampon (Asp, Thr, Ser, Gly, Ala, Val). Le fait que des protéines peuvent être transférées à partir d'une feuille de gel et être immobilisées sur plusieurs feuilles superposées de verre poreux revêtues de POLYBRENE, indique que les capacités de fixation de protéines sont déterminées, pour un type donné de feuille poreuse en verre revêtu du film du matériau utilisé, ne serait-ce qu'en raison de la capacité maximum d'absorption de protéines.

Les capacités de fixation par une feuille de fibre de verre de diverses protéines ont été déterminées pour plusieurs protéines en mesurant les quantités de protéines qui étaient retenues dans des régions saturées des feuilles (les régions de la première feuille correspondant à celles de la deuxième feuille, etc.).

Les résultats ont été ensuite normalisés en pg de protéine par cm2 et sont résumés dans le tableau ci-après. Les capacités maximales ont été déterminées pour la myoglobine de muscle squelettique de cachalot, la phosvitine d'oeuf de poulet, l'actine de muscle squelettique de lapin, le chymotrypsinogène bovin, la sérumalbumine humaine et la DNase I pancréatique bovine.

Les protéines ont été déposées sur le gel en quantités suffisantes pour permettre une analyse amino-acide précise (de 1 nmol à 300 pmol). Les caractéristiques de transfert de ces protéines sont montrées à la figure 3. Les capacités de fixation ont été mesurées soit après le transfert direct et la détection avec la fluorescamine (Tableau 1, ligne 1) soit après la détection avec le bleu de Coomassie suivie du transfert (Tableau 1, ligne 2).

Les valeurs données à la ligne 1 sont des mo₋ yennes de deux déterminations indépendantes, chacune d'entre-elles ne différant pas de la valeur moyenne de plus de 10%.

Ceci indique que la capacité de transfert pour chaque protéine est relativement constante, indépendante de l'expérience. Les valeurs ont été basses pour la phosvitine (protéine phosphorylatée) (8 pg/cm2) et variables pour les autres protéines entre 15 et 28 pg/cm2.

La capacité de fixation pour les protéines colorées avec le bleu de Coomassie (ligne 2) a été identique aux valeurs ci-dessus pour 3 protéines sur 6, mais seulement égale à la moitié pour la myoglobine et l'actine et extrêmement basse pour la phosvitine. De plus, ces dernières valeurs ont varié d'une manière sensible d'une expérience à l'autre (+ de 50%). Comme ci-dessus, les valeurs données à la ligne 2 du Tableau 1 représentent des moyennes de deux expériences indépendantes.

Cette variabilité sensible semble être due à une saturation incomplète du gel avec le tampon SDS- borate avant l'électrotransfert.

- La figure 4 A montre le résultat de la coloration de différentes quantités de sérumalbumine humaine (de 5 à 500 ng) avec le bleu de Coomassie,
- La figure 4C la coloration avec l'argent (20)
- La figure 4B avec la fluorescamine, cette dernière étant effectuée après un transfert électrophorétique sur le verre recouvert de POLYBRENE. Les résultats démontrent que pour la sérumalbumine, la détection avec la fluorescamine (en utilisant 1 mg/200 ml) présente une meilleure sensibilité que celle avec le bleu de Coomassie, mais moins bonne qu'avec l'argent.

### DETERMINATION DIRECTE DE LA COMPOSITION DES SEOUENCES D'ACIDES AMINES PAR UN SEOUENCAGE EN PHASE GAZEUSE.

Une expérience a été réalisée avec 20 pg 1,7 nmol) de myoglobine de muscle de squelette de cachalot, lequel avait auparavant subi une électrophorèse sur gel de polyacrylamide-SDS avant d'être immobilisé sur du verre recouvert de POLYBRENE.

La protéine immobilisée a été soumise à 18 cycles de la dégradation d'Edman et la quantité ainsi que la nature des acides aminés-PTH mis en évidence après chaque étape ont été déterminées par une analyse CLHP.

Ces données ont permis de déterminer les efficacités de séquençage. Le rendement de couplage initial (30_{%}) a été calculé en comparant la quantité de myoglobine immobilisée et transférée (1 nmole) et le rendement de l'acide aminé PTH correspondant à l'acide aminé NH₂- terminal (300 pmol).

Les rendements répétés ont été calculés à partir des rendements de la leucine PTH en positions 2, 9 et 11 et ceux de la Valine PTH en positions 1, 10 et 13; des valeurs de 91% et 92% ont été trouvées respectivement.

Un séquençage semblable en phase gazeuse a été effectué avec la même quantité de myoglobine (1 nanomo- le) qui avait été directement déposée sur un disque de fibre de verre recouvert auparavant avec 30 µl d'une solution de POLYBRENE (100 mg/ml).

Le second séquençage a été effectué de manière classique. Il a montré un rendement initial supérieur (55%) mais les rendemants répétés ont été similaires à la première expérience.

Le séquençage en phase gazeuse de protéines immobilisées sur du verre recouvert de POLYBRENE ne conduit à la production que de très faibles quantités de diphénylthiourée (DPTU) et de diphénylurée (DPU), qui constituent des produits secondaires de la dégradation d'Edman, normalement détectés en grande quantité dans les chromatogrammes CLHP des acides aminés PTH. L'absence de ces produits secondaires est un avantage spécial puisqu'elle permet une identification certaine de la PTH-Met et PTH-Ile, éluant dans le système de séparation de HUNKAPILLER et HOOD (17).

Le procédé de transfert selon l'invention fournit donc des moyens permettant un séquençage "in situ" particulièrement efficace et reproductible de quantités initialement excessivement faibles de protéines. Ci-après est encore fournie la bibliographie des articles qui doivent être considérés comme faisant partie de la présente description, dans la mesure où ils seraient utiles à une meilleure compréhension de son contenu.

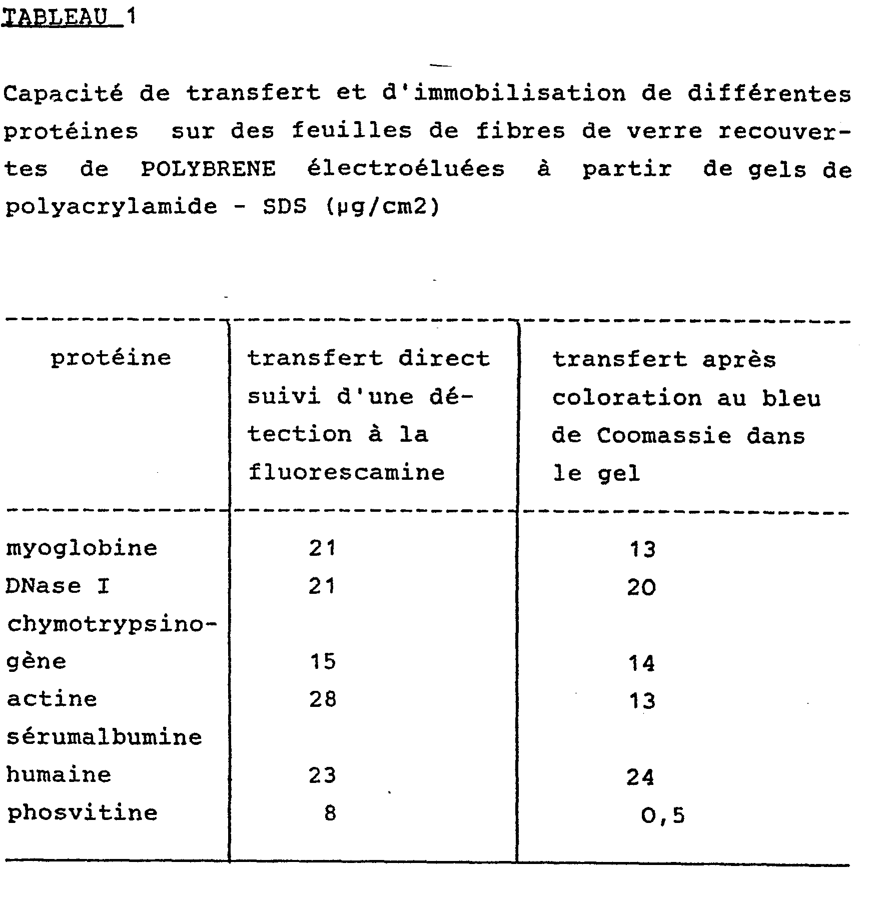

## Revendications

1/ Procédé de transfert et d'immobilisation de protéines et/ou d'acides nucléiques sur une surface libre formée sur un support chimiquement inerte, de préférence poreux, à partir d'un milieu susceptible de les fournir, amené en contact avec cette surface, caractérisé en ce que ladite surface a un caractère généralement hydrophobe tout en portant des charges d'un signe déterminé et en ce que les protéines et/ou les acides nucléiques sont amenées à l'état solubilisé au contact de ladite surface, en présence d'un détergent portant des charges d'un signe opposé au signe déterminé, la concentration du détergent étant suffisante pour, si besoin, maintenir la solubilité desdites protéines et/ou des acides nucléiques dans le milieu, mais ne dépassant pas celle qui entraînerait une fixation trop sélective du détergent à ladite surface au détriment des protéines et/ou des acides nucléiques.

2/ Procédé selon la revendication 1, caractérisé en ce que le support chimiquement inerte est choisi dans le groupe comportant notamment le verre, de préférence sous forme de fibres, les polymères de l'acide méthacrylique, les copolymères de l'acide méthacrylique et de l'éthylène, des copolymères du chlorure de vinyle et de l'acétate de vinyle, les polymères de l'acide cis-oléique.

3/ Procédé selon la revendication 1 ou 2, caractérisé en ce que le milieu à partir duquel les protéines et/ou les acides nucléiques sont transférées vers ladite surface est essentiellement formé par une feuille d'un gel, notamment à base de polyacrylamide, amené au contact de la surface libre.

4/ Procédé selon la revendication 3, caractérisé en ce que le gel porte plusieurs bandes de protéines et/ou d'acides nucléiques et en ce que le transfert est effectué par élution des protéines et/ou des acides nucléiques hors du gel et vers la surface libre du support.

5/ Procédé selon l'une quelconque des revendications 1 à 4, caractérisé en ce que la surface libre est formée sur une pellicule hydrophobe immobilisée à la surface du support inerte par l'intermédiaire de liaisons assurant son insolubilisation vis-à-vis de solutions aqueuses et organiques venant à son contact.

6/ Procédé selon la revendication 5, caractérisé en ce que la pellicule est formée d'un produit POLYMERE généralement hydrophobe isolant le support du milieu contenant les protéines et/ou les acides nucléiques et amenée à son contact, ladite pellucule comportant des groupes chargés positivement affleurant à sa surface extérieure et en ce que le détergent utilisé comporte des charges négatives.

7/ Procédé selon la revendication 6, caractérisé en ce que la pellicule est formée d'un produit comportant des chaînes hydrocarbonées hydrophobes, dans lesquelles sont intercalées des groupes ammonium quaternaire.

8/ Procédé selon la revendication 7, caractérisé en ce que le produit formant la pellicule est choisi dans le groupe comportant notamment le polymé- _ thobromure de 1,5 diméthyl-1,5 diazaundécaméthylène, les sels de poly(2-vinyl-N-méthyl pyridine), les sels de poly(4-vinyl-N-méthylpyridine).

9/ Procédé selon la revendication 5 prise isolément ou en combinaison avec l'une quelconque des revendications 6 à 8, caractérisé en ce que le détergent est constitué de dodécylsulfate de sodium.

10/ Procédé selon la revendication 1 ou 2, caractérisé en ce que ladite surface porte des groupes chargés négativement et en ce que le détergent porte des groupes chargés positivement.

11/ Procédé selon la revendication 10, caractérisé en ce que le support chimiquement inerte est constitué par du verre poreux et en ce que le détergent au contact duquel les protéines et/ou les acides nucléiques sont amenées au contact de la surface libre du support porte des groupes chargés positivement, et en ce que le transfert est fait à la surface du verre directement.

12/ Procédé selon la revendication 11, caractérisé en ce que le détergent est constitué de bromure de céthyl-triméthyl ammonium.

_{13/} Procédé selon la revendication 3 ou 4, elle-même combinée avec l'une quelconque des revendications 5 à 12, caractérisé en ce que le gel, notamment de polyacrylamide, renfermant les protéines et/ou les acides nucléiques contient une concentration en détergent suffisante à maintenir et à assurer la solubilisation des protéines et/ou des acides nucléiques pendant l'opération de transfert.

14/ Procédé. selon la revendication 13, caractérisé en ce que le transfert est réalisé en mettant en oeuvre une solution éluante dépourvue de détergent.

15/ Pellicule immobilisée et insolubilisée vis-à-vis de solutions aqueuses ou organiques par liaison à la surface d'un support chimiquement inerte, comportant des groupes chargés négativement, ladite pellicule étant formée d'un produit comportant à la fois des groupes hydrophobes et des groupes chargés positivement affleurant à sa surface libre, ce produit étant de préférence dépourvu de protéines et/ou d'acides nucléiques.

_{16/} Pellicule selon la revendication 15, caractérisé en ce qu'elle est immobilisée à la surface d'un support chimiquement inerte, choisi dans le groupe comportant notamment le verre, de préférence sous forme de fibres, les polymères de l'acide méthacrylique, les copolymères de l'acide méthacrylique et de l'éthylène, les copolymères du chlorure de vinyle et de l'acétate de vinyle, les polymères de l'acide cis-oléique.

17/ Pellicule supportée selon la revendication 16, caractérisée en ce qu'elle est formée par un produit polymère hydrophobe comprenant des chaînes hydrocarbonées dans lesquelles sont intercalées des groupes ammonium quaternaire formant des charges positives dont une partie au moins affleure à la surface libre de la pellicule.

18/ Pellicule supportée selon la revendication 17, caractérisée en ce qu'elle est formée d'un produit choisi dans le groupe comportant notamment le polyméthobromure de 1,5 diméthyl-1,5 diazaundécaméthylène, les sels de poly(2-vinyl-N-méthylpyridine), les sels de poly(4-vinyl-N-méthylpyridine), le produit étant ioniquement fixé au support.

19/ Utilisation de la pellicule insolubilisée selon l'une quelconque des revendications 15 à 18 pour la détection des anticorps contenus dans un échantillon biologique, par réaction avec les protéines antigéniques complémentaires préalablement fixées et immobilisées sur ladite pellicule insolubilisée.

20/ Procédé de formation d'une pellicule insolubilisée vis-à-vis de solvants aqueux ou organiques sur un support chimiquement inerte, portant des groupes chargés négativement, caractérisé par la mise en contact de la surface de ce support avec une solution aqueuse ou organique, d'une matière dont la structure moléculaire comporte à la fois des régions hydrophobes et des groupes chargés positivement permettant sa solubilisation, le nombre de groupes chargés positivement et la concentration de la matière dans la solution étant suffisants à permettre la neutralisation des charges négatives portées par le support, ainsi que la formation et la rétention d'une pellicule au moins monomoléculaire hydrophobe portant un excédent de charges positives à sa surface, après rinçage et séchage dudit support revêtu de la pellicule insolubilisée alors formée.
